(19) Europäisches Patentamt
European Patent Office
Office européen des brevets

(11) EP 0 623 340 B1

(12) **FASCICULE DE BREVET EUROPEEN**

(45) Date de publication et mention de la délivrance du brevet: **17.09.1997 Bulletin 1997/38**

(51) Int. Cl.$^6$: **A61K 7/48**

(21) Numéro de dépôt: **94400957.0**

(22) Date de dépôt: **03.05.1994**

(54) **Utilisation d'hydroxy ou de dihydroxy naphtalènes dans la préparation d'une composition cosmétique ou dermatologique à action dépigmentante**

Verwendung von Hydroxy- oder Dihydroxynaphthalinen zur Bereitung einer kosmetischen oder dermatologischen Zusammensetzung zur Hautdepigmentierung

Use of hydroxy or dihydroxynaphthalene in the preparation of a cosmetic or dermatological composition for skin whitening

(84) Etats contractants désignés:
**DE ES FR GB IT**

(30) Priorité: **03.05.1993 FR 9305233**

(43) Date de publication de la demande:
**09.11.1994 Bulletin 1994/45**

(73) Titulaire: **L'OREAL**
**75008 Paris (FR)**

(72) Inventeur: **Lagrange, Alain**
**F-77700 Coupvray (FR)**

(74) Mandataire: **Stalla-Bourdillon, Bernard**
**CABINET NONY & CIE**
**29, rue Cambacérès**
**75008 Paris (FR)**

(56) Documents cités:
**GB-A- 1 028 923** **US-A- 3 415 608**

- **PATENT ABSTRACTS OF JAPAN vol. 17, no. 426 (C-1094)9 Août 1993 & JP-A-50 092 916 (SANSHO SEIYAKU CO LTD) 5 Mars 1991**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.14.14/3.4

## Description

La présente invention a pour objet l'utilisation d'hydroxy ou de dihydroxy naphtalènes dans des compositions cosmétiques ou dermatologiques par application topique dans le but de blanchir la peau ou de traiter les taches pigmentaires.

On rappellera que le mécanisme de formation de la pigmentation de la peau, c'est-à-dire de la formation des mélanines est particulièrement complexe et fait intervenir schématiquement les principales étapes suivantes :

Tyrosine → Dopa → Dopaquinone → Dopachrome → Mélanines

l'enzyme intervenant dans cette suite de réactions étant essentiellement la tyrosinase.

Les substances les plus utilisées à l'heure actuelle en tant que dépigmentants sont plus particulièrement l'hydroquinone et ses dérivés en particulier ses éthers tels que le monométhyléther d'hydroquinone.

Ces composés, s'ils ont une efficacité certaine, ne sont malheureusement pas exempts d'effets secondaires, ce qui peut rendre leur emploi délicat voir dangereux.

Ainsi l'hydroquinone, dont l'emploi est d'ailleurs limité à une concentration de 2 %, est un composé particulièrement irritant et cytotoxique pour le mélanocyte, dont le remplacement, total ou partiel a été envisagé par de nombreux auteurs.

Il a ainsi été proposé dans le brevet US n° 4 526 179 certains esters gras d'hydroquinone ayant une bonne activité et étant moins irritants et plus stables que l'hydroquinone.

De même on a proposé d'autres dérivés d'hydroquinone dans la demande japonaise n° 27909/86 ne présentant pas les inconvénients de l'hydroquinone mais dont l'efficacité s'est révélée relativement médiocre.

Il est bien établi qu'une substance exerce une action dépigmentante si elle agit directement sur la vitalité des mélanocytes épidermiques où se déroule normalement la mélanogénèse, et/ou si elle interfère avec une des étapes de la biosynthèse des mélanines soit en inhibant un des enzymes impliqués soit en s'intercalant comme analogue structural dans la voie de synthèse qui peut ainsi être bloquée d'où l'effet dépigmentant.

L'utilisation de substances dépigmentantes topiques présentant une bonne efficacité et étant inoffensive, est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou secondaires à la contraception oestro-progestative, les hyperpigmentations localisées par hyperactivité et prolifération mélanocytaire bénigne telles que les taches pigmentaires séniles dites lentigo actiniques, les hyperpigmentations accidentelles telles que la photosensibilisation et la cicatrisation post-lésionnelle, ainsi que certaines leucodermies telles que le vitiligo où, à défaut de pouvoir repigmenter la peau lésée, on achève de dépigmenter les zones de peau normale résiduelle pour donner à l'ensemble de la peau une teinte blanche homogène.

Après de nombreuses études sur différentes substances on a constaté de façon tout à fait surprenante que certains hydroxy ou dihydroxy naphtalènes avaient une action dépigmentante, qui pour la plupart, s'est avérée être supérieure à celle de l'hydroquinone dans le test d'inhibition "in vitro" de l'activité de la tyrosinase comme ceci sera décrit ci-après.

La présente invention a donc pour objet l'utilisation à titre de composés actifs d'hydroxy ou de dihydroxy naphtalènes pour la préparation d'une composition cosmétique ou dermatologique ayant une action dépigmentante, les dits composés répondant à la formule générale suivante :

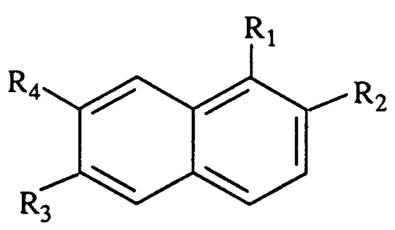

(I)

dans laquelle :

$R_1$ à $R_4$ représentent un atome d'hydrogène ou un radical hydroxyle, l'un au moins et deux au plus des radicaux $R_1$ à $R_4$ représentant un radical hydroxyle, à l'exception du 1 -hydroxynaphtalène.

Parmi les composés de formule (I), on peut citer notamment les composés suivants :

- le 2-hydroxynaphtalène,
- le 1,6-dihydroxynaphtalène,

- le 1,7-dihydroxynaphtalène,
- le 2,6-dihydroxynaphtalène, et
- le 2,7-dihydroxynaphtalène.

Parmi les composés particulièrement préférés, on peut citer notamment :

- le 2,6-dihydroxynaphtalène, et
- le 1,6-dihydroxynaphtalène.

Dans les compositions dépigmentantes selon l'invention, la concentration en composés de formule (I) est généralement comprise entre 0,01 % et 10 % et de préférence entre 0,5 % et 5 % en poids par rapport au poids total de la composition.

Le véhicule des compositions peut être notamment une solution aqueuse ou hydroalcoolique, une émulsion du type huile-dans-l'eau ou eau-dans-l'huile, un gel émulsionné ou encore un système biphasé.

De préférence les compositions selon l'invention se présentent sous forme d'une lotion, d'une crème, d'un lait, d'un gel, d'un masque, de microsphères ou nanosphères ou de dispersions vésiculaires. Dans le cas des dispersions vésiculaires, les lipides constitutifs des vésicules peuvent être du type ionique ou non ionique ou bien un mélange de ceux-ci.

Ces compositions cosmétiques peuvent également contenir un humectant, un agent de surface, un kératolytique, un agent anti-inflammatoire, un agent complexant, un anti-oxydant, un conservateur, un parfum ou un filtre solaire.

Ces compositions sont appliquées par voie topique en quantité correspondant aux doses d'application usuelles pour le type de composition considéré (gel, crème, lotion, etc...). Par exemple dans le cas d'une crème on utilise de 0,5 à 3 mg et notamment de 1 à 2 mg de crème par cm2 de peau et par application, à raison d'une ou deux applications par jour.

Les composés de formule générale (I) sont connus et peuvent être préparés selon les méthodes conventionnelles.

Etudes "in vitro"

Certains des composés de formule générale (I) ont été étudiés comparativement à l'hydroquinone à quantité molaire équivalente dans le test d'inhibition in vitro de l'activité de la tyrosinase.

Selon ce test on suit par spectrométrie visible à 475 nm la quantité de dopachrome formée au cours de la chaîne de réactions de transformation de la tyrosine en mélanines. Ces réactions sont catalysées in vitro par la tyrosinase de champignons, en présence d'un co-substrat réducteur (par exemple la L-dopa en petite quantité) pour initier la réaction d'hydroxylation de la L-tyrosine en L-dopa, laquelle est ensuite oxydée catalytiquement en dopaquinone puis en dopachrome, produit intermédiaire avant les réactions d'oxydation non enzymatiques aboutissant à la formation des mélanines.

On mesure donc la concentration en dopachrome formé au cours du temps en présence et en absence de l'inhibiteur.

Les concentrations en inhibiteurs sont fixées à 50 % molaire par rapport à la concentration en tyrosine dans le milieu réactionnel.

On exprime l'effet d'inhibition par l'abaissement de la quantité maximale de dopachrome formée (valeur de densité optique à 475 nm lue au plateau de la courbe) par rapport à la quantité obtenue en l'absence d'inhibiteur.

*Protocole expérimental*

*Réactifs :*

A - Tampon phosphate 0,1 M pH = 6,5 (Tween 20 à 1 %)
B - Solution mère de L-tyrosine à $2.10^{-3}$ M dans A
C - Solution mère de L-dopa à $10^{-4}$ M dans A
D - Solution mère de tyrosinase de champignons à 2.400 unités/ml dans A
E - Solution mère de l'inhibiteur à $10^{-2}$ M dans A (les solutions C et D sont à préparer le jour même)

*Résultats :*

- cuve de référence : 3 ml de A
- cuve d'essai :
  1 ml de B
  0,1 ml de C

1,85ml de A + E

- homogénéiser et équilibrer à 25°C
- ajouter 0,05 ml de D
- mélanger rapidement et observer la cinétique par la mesure de l'absorbance à 475 nm en fonction du temps.

TABLEAU I

| *Composés* | *% d'inhibition* |
|---|---|
| 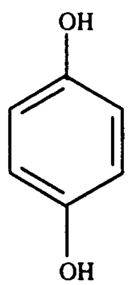 (hydroquinone) | -33 % |
| 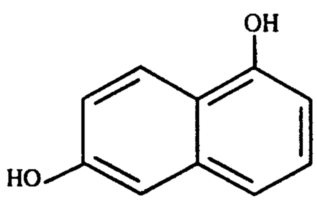 | -47 % |
| 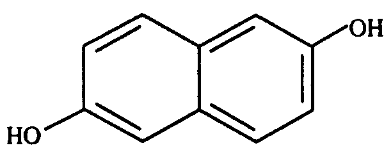 | -91 % |

EXEMPLES DE COMPOSITION

**EXEMPLE 1 :***Crème dépigmentante*

| | |
|---|---|
| - 2,6-dihydroxynaphtalène | 1g |
| - Alcool cétylstéarylique oxyéthyléné à 12 moles d'oxyde d'éthylène | 0,5 g |
| - Alcool cétylstéarylique oxyéthyléné à 20 moles d'oxyde d'éthylène | 0,5 g |
| - Monostéarate de glycol | 3 g |
| - Mélange de caprylate et caprate de coprah | 5 g |
| - Polymère d'acide acrylique réticulé vendu sous la dénomination "Carbomer® 934P" par la Société Goodrich | 0,3 g |
| - Triéthanolamine | 0,9 g |
| - Ethanol | 25 g |
| - Parfum, conservateurs, q.s. | |
| - Eau q.s.p. | 100 g |

**EXEMPLE 2**: *Lotion dépigmentante*

| | |
|---|---|
| - 2,6-dihydroxynaphtalène | 3 g |
| - Ethanol | 50 g |
| - Polyéthylèneglycol 400 | 30 g |
| - Ethoxy diglycol | 5 g |
| - Glycérine | 5 g |
| - Eau q.s.p. | 100 g |

Dans cet exemple, le 2,6-dihydroxynaphtalène peut être remplacé par 2 g de 1 ,6-dihydroxynaphtalène.

**EXEMPLE 3** : *Crème dépigmentante*

| | |
|---|---|
| - 2,6-dihydroxynaphtalène | 0,5 g |
| - Propylène glycol | 10 g |
| - Huile de vaseline | 20,5 g |
| - Isostéarate de sorbitan | 5 g |
| - Mélange de caprylate et caprate de coprah | 1 g |
| - Mélange d'hectorite modifiée et de carbonate de propylène vendu sous la dénomination de "Miglyol® Gel" par la Société Dynamit Nobel | 5 g |
| - Eau q.s.p. | 100 g |

## Revendications

1. Utilisation à titre de composés actifs d'hydroxy ou de dihydroxy naphtalènes pour la préparation d'une composition cosmétique ou dermatologique ayant une action dépigmentante, caractérisée par le fait que les dits composés répondent à la formule suivante :

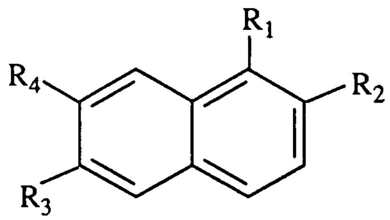

(I)

dans laquelle :
$R_1$ à $R_4$ représentent un atome d'hydrogène ou un radical hydroxyle, l'un au moins et deux au plus des radicaux $R_1$ à $R_4$ représentant un radical hydroxyle, à l'exception du 1 -hydroxynaphtalène.

2. Utilisation selon la revendication 1, caractérisée par le fait que lesdits composés de formule (I) sont choisis dans le groupe constitué par :

   - le 2-hydroxynaphtalène,
   - le 1,6-dihydroxynaphtalène,
   - le 1,7-dihydroxynaphtalène,
   - le 2,6-dihydroxynaphtalène, et
   - le 2,7-dihydroxynaphtalène.

3. Utilisation selon l'une quelconque des revendications précédentes, caractérisée par le fait que lesdits composés de formule (I) sont choisis de préférence parmi le 2,6-dihydroxynaphtalène et le 1,6-dihydroxynaphtalène.

4. Utilisation selon l'une quelconque des revendications précédentes, caractérisée par le fait que la concentration en composés de formule (I) dans la composition est comprise entre 0,01 % et 10 % en poids par rapport au poids total de la composition.

5. Utilisation selon l'une quelconque des revendications précédentes, caractérisée par le fait que la concentration en composés de formule (I) dans la composition est de préférence comprise entre 0,5 % et 5 % en poids par rapport au poids total de la composition.

6. Utilisation selon l'une quelconque des revendications précédentes caractérisée par le fait que la composition cosmétique ou dermatologique se présente sous forme d'une lotion, d'une crème, d'un lait, d'un gel, d'un masque, de microsphères ou nanosphères ou de dispersions vésiculaires.

7. Utilisation selon l'une quelconque des revendications précédentes, caractérisée par le fait que la composition cosmétique ou dermatologique contient en outre un humectant, un agent de surface, un kératolytique, un agent anti-inflammatoire, un agent complexant, un anti-oxydant, un conservateur, un parfum ou un filtre solaire.

## Claims

1. Use, as active compounds, of hydroxy- or dihydroxynaphthalenes for the preparation of a cosmetic or dermatological composition having a depigmenting action, characterized by the fact that the said compounds correspond to the following formula:

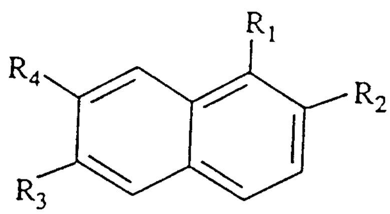

(I)

in which:
$R_1$ to $R_4$ represent a hydrogen atom or a hydroxyl radical, at least one and at most two of the radicals $R_1$ to $R_4$ representing a hydroxyl radical, with the exception of 1-hydroxynaphthalene.

2. Use according to Claim 1, characterized by the fact that the said compounds of formula (I) are chosen from the group consisting of:

- 2-hydroxynaphthalene,
- 1,6-dihydroxynaphthalene,
- 1,7-dihydroxynaphthalene,
- 2,6-dihydroxynaphthalene, and
- 2,7-dihydroxynaphthalene.

3. Use according to any one of the preceding claims, characterized by the fact that the said compounds of formula (I) are preferably chosen from 2,6-dihydroxynaphthalene and 1,6-dihydroxynaphthalene.

4. Use according to any one of the preceding claims, characterized by the fact that the concentration of compounds of formula (I) in the composition is between 0.01% and 10% by weight relative to the total weight of the composition.

5. Use according to any one of the preceding claims, characterized by the fact that the concentration of compounds of formula (I) in the composition is preferably between 0.5% and 5% by weight relative to the total weight of the composition.

6. Use according to any one of the preceding claims, characterized by the fact that the cosmetic or dermatological composition is provided in the form of a lotion, a cream, a milk, a gel, a face-pack, microspheres or nanospheres or vesicular dispersions.

7. Use according to any one of the preceding claims, characterized by the fact that the cosmetic or dermatological composition contains, in addition, a humectant, a surfactant, a keratolytic agent, an anti-inflammatory agent, a complexing agent, an antioxidant, a preservative, a perfume or a sunscreen agent.

**Patentansprüche**

1. Verwendung von Hydroxy- oder Dihydroxynaphthalin-Wirkstoffen zur Herstellung einer kosmetischen oder dermatologischen Zubereitung mit einer depigmentierenden Wirkung, dadurch gekennzeichnet, daß diese Verbindungen der folgenden Formel entsprechen:

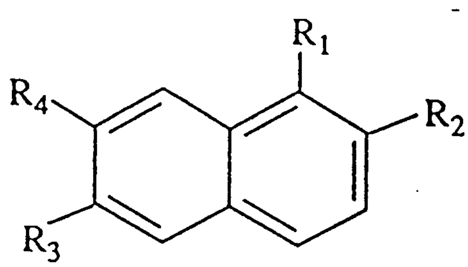

(I)

worin
$R_1$ bis $R_4$ ein Wasserstoffatom oder eine Hydroxygruppe bedeuten, wobei mindestens einer oder höchstens zwei der Reste $R_1$ bis $R_4$ eine Hydroxygruppe mit Ausnahme des 1-Hydroxynaphthalins darstellen.

**2.** Verwendung nach Anspruch 1, dadurch gekennzeichnet, daß die Verbindungen gemäß der Formel (I) aus der aus

2-Hydroxynaphthalin,
1,6-Dihydroxynaphthalin,
1,7-Dihydroxynaphthalin,
2,6-Dihydroxynaphthalin, und
2,7-Dihydroxynaphthalin

bestehenden Gruppe ausgewählt sind.

**3.** Verwendung nach einem der vorstehenden Ansprüche, dadurch gekennzeichnet, daß die Verbindungen gemäß der Formel (I) vorzugsweise aus 2,6-Dihydroxynaphthalin und 1,6-Dihydroxynaphthalin ausgewählt sind.

**4.** Verwendung nach einem der vorstehenden Ansprüche, dadurch gekennzeichnet, daß die Konzentration an Verbindungen gemäß der Formel (I) in der Zubereitung zwischen 0,01 Gew.-% und 10 Gew.-% in bezug auf das Gesamtgewicht der Zusammensetzung beträgt.

**5.** Verwendung nach einem der vorstehenden Ansprüche, dadurch gekennzeichnet, daß die Konzentration an Verbindungen gemäß der Formel (I) in der Zubereitung vorzugsweise zwischen 0,5 Gew.-% und 5 Gew.-% in bezug auf das Gesamtgewicht der Zusammensetzung beträgt.

**6.** Verwendung nach einem der vorstehenden Ansprüche, dadurch gekennzeichnet, daß die kosmetische oder dermatologische Zubereitung in Form einer Lotio, Creme, Milch, eines Gels, einer Maske, in Form von Mikrokügelchen oder Nanokügelchen oder in Form von vesikulären Dispersionen vorliegt.

**7.** Verwendung nach einem der vorstehenden Ansprüche, dadurch gekennzeichnet, daß die kosmetische oder dermatologische Zubereitung zusätzlich noch ein Feuchthaltemittel, Tensid, Keratolytikum, einen Entzündungshemmer, ein Komplexiermittel, Antioxidans, Konservierungsmittel, einen Duftstoff oder eine Sonnenfiltersubstanz enthält.